# EUROPEAN PATENT SPECIFICATION

(11) **EP 3 316 819 B1**
(45) Date of publication and mention of the grant of the patent: **11.11.2020**
(21) Application number: 16740981.2
(22) Date of filing: 04.07.2016
(51) Int. Cl.: A61F 2/24

(54) **BALLOON CATHETER FOR PROSTHETIC VALVES**
BALLONKATHETER FÜR KLAPPENPROTHESEN
CATHÉTER À BALLONNET POUR VALVULES PROTHÉTIQUES

(30) Priority: 03.07.2015 EP 15175364
(43) Date of publication of application: 09.05.2018
(73) Proprietor: Symetis SA, 1024 Ecublens (CH)
(72) Inventor: DELALOYE, Stéphane, 8180 Bulach (CH)
(74) Representative: Peterreins Schley
(86) International application number: PCT/EP2016/065680
(87) International publication number: WO 2017/005683

(56) References cited:
- WO-A1-97/40877
- US-A1- 2011 264 201

## Description

### Field of the Disclosure

The present disclosure relates to the field of balloon catheters for use with expandable prosthetic valves for transcatheter delivery. In one non-limiting aspect, the disclosure relates to a balloon catheter configured for applying expansion forces to a prosthetic valve after the valve has been implanted (so called post-implantation dilation). In another closely related, but non-limiting aspect, the disclosure relates to a balloon catheter configured for a cardiac prosthetic valve.

### Background to the Invention

Transcatheter delivery of an expandable cardiac valve is a minimally invasive and/or percutaneous technique in which a replacement cardiac valve (or stent-valve) is delivered in a collapsed condition to a site of implantation using a catheter, and expanded by or from the delivery catheter to an expanded condition for implantation.

A so-called balloon-expandable stent-valve is designed to be expanded by a dedicated deployment balloon on the delivery catheter. The stent-valve is crimped around the balloon for delivery. Inflation of the balloon causes plastic deformation of the stent to embed the stent into the tissue at the implantation site. The stent has a high crush resistance, so that once the deployment balloon is deflated, the stent remains embedded and does not dislodge from its implanted position in use.

A so-called self-expandable stent-valve includes an elastic or superelastic stent that self-expands from a collapsed condition towards an expanded condition, without needing a deployment balloon. The stent is oversized with respect to the implantation site, meaning that the stent does not expand to its full size, but instead is constrained by the implantation site and applies an outward expansion force on the surrounding tissue to hold the stent in position at the implantation site.

Although a self-expandable stent-valve does not require a deployment balloon to expand, occasionally a medical practitioner performs a so-called post implantation dilation procedure, in which a dilation balloon is inserted and inflated after the self-expanding stent-valve has been implanted in the usual way (self-expansion). Such a procedure is different from balloon expansion described above. Instead of plastically or artificially expanding a self-expanding stent, the purpose of post-implantation dilation is to force the tissue surrounding the prosthetic valve to model better around the self-expanding frame, for example to reduce para-valve leakage, while keeping the stent in its self-expanding size-range.

Balloon catheters with regions of different diameters are known e.g. from WO 97/40877 and US 2011/0264201.

### Summary of the Disclosure

The present invention is defined by the appended claims.

Aspects, embodiments or examples of the present disclosure which do not fall within the scope of the appended claims are not part of the present invention.

The following presents a non-limiting, simplified summary in order to provide a basic understanding of some aspects of the disclosure. This summary is not an extensive overview of the disclosure. It is intended to neither identify key or critical elements of the disclosure nor delineate the scope of the disclosure. Its sole purpose is to present some concepts of the disclosure in a simplified form as a prelude to the more detailed description that is presented later.

In one aspect, there is disclosed herein a balloon catheter with an inflatable region, and intended for post-implantation dilation of a stent-valve. The balloon catheter may any have one or a combination of any two or more of the following features, which are all optional:
(a) the inflatable region may include a first region of first diameter and first axial length, and a second region neighboring the first region, the second region of second diameter and second axial length, the second diameter being smaller than the first diameter. Optionally, at least one, and optionally both, of the first and second regions has or have a cylindrical shape.
(b) the inflatable region may be shaped for applying a dilation force to an anchor portion of the stent-valve (or stent component), while applying less dilation to a leaflet portion of the stent-valve (or valve component). Such a technique may reduce the potential risk of damage to the leaflet portion, especially when used by a less-experienced practitioner.
(c) the inflatable region may further include a third region neighboring the first region on an opposite side to the second region, the third region of third diameter and third axial length, the third diameter smaller than the first diameter. Optionally the third region has a cylindrical shape. All references below to the third region, or third axial length, or third diameter are intended to be optional, intended to be read only if the third region is provided, without implying that the third region is necessarily provided.
(d) A fourth region of decreasing diameter may extend away from the second region, and/or a fifth region of decreasing diameter may extend away from the third region.
(e) A difference between the first diameter, and at least one of the second and third diameters, may optionally be (i) between about 1mm and about 5mm; (ii) optionally between about 1mm and about 3mm; (iii) optionally between about 1mm and about 2mm.
(f) The second and third diameters may be about equal, or they may be different from each other. Additionally or alternatively, the second and third axial lengths may be about equal, or they may be different from each other.
(g) The first axial length may optionally not be smaller than at least one, optionally both, of the second and third axial lengths individually. Additionally or alternatively, the first axial length may optionally be about equal to the sum of the second and third axial lengths.
(h) The first axial length may be between about 10mm and about 30mm.
(i) At least one of the second and third axial lengths may be between about 5mm and about 20mm.
(j) The first, second and third regions may define a generally continuous internal inflation space of the inflatable region.
(k) An interface region may be defined between the neighboring first and third regions, and/or between the neighboring first and third regions. The interface region may bridge a diameter difference between the respective regions.
(l) If provided, the interface region may have a relative short axial length (or axial extent), for example, less than about 5mm, optionally less than about 4mm, optionally less than about 3mm, optionally less than about 2mm, optionally less than about 1mm. Additionally or alternatively, the axial length of the interface region may optionally be not greater than the diameter difference bridged by the interface region. Additionally or alternatively to any of the foregoing, the interface region may correspond to a step-change in diameter.
(m) The first region may be a longitudinally central region of the inflatable region.
(n) The first region may correspond to a maximum diameter of the inflatable region. It will be appreciated that the first diameter (of the first region) may be depend on the intended implantation size desired by the medical practitioner. The balloon catheter may be provided in a range of predetermined sizes, enabling the practitioner to select a specific balloon catheter of a size to match the implantation site, and/or to match the size of the implanted stent-valve.
(o) The balloon catheter may be configured for post-implantation dilation of a stent-valve that comprises a stent component and a valve component, the balloon catheter comprising an inflatable region shaped for applying a dilation force to an anchor portion of the stent component, while applying less dilation to a leaflet portion of the valve component.
(p) The inflatable region may have a shape including any one or more of the following characteristics, which are all optional:
   - generally non-cylindrical overall shape;
   - generally non-dog-bone overall shape;
   - a shape including a larger diameter region (e.g. first region) configured for applying a dilation force to the anchor portion of the stent-component, and a smaller diameter region (e.g. second or third region) for applying less dilation to a leaflet portion of the valve component.
      - Optionally the difference in diameter may be at least about 1mm, or at least about 2mm, or at least about 3mm, or at least about 4mm, or at least about 5mm.
      - Additionally or alternatively to any of the above, the difference in diameter may be not more than about 5mm, or not more than about 4mm, or not more than about 3mm, or not more than about 2mm, or not more than about 1mm.
      - Additionally or alternatively to any of the above, the larger diameter region may be generally cylindrical in shape or at least include a generally cylindrically shaped region.
      - Additionally or alternatively to any of the above, the smaller diameter region may be generally cylindrical in shape or at least include a generally cylindrically shaped region.
      - Additionally or alternatively to any of the above, the larger and smaller diameter regions may be generally contiguous with each other, and joined at a step-change in the diameter of the inflatable region.
      - Additionally or alternatively to any of the above, the shape may further comprise a second smaller diameter region of diameter smaller than the larger diameter region. The first and second smaller diameter regions may optionally be of about the same diameter. The first and second smaller diameter regions may be arranged on opposite ends of the inflatable region and/or on opposite axial sides of the larger diameter region.
(q) The inflatable region may communicate with an inflation lumen defined by a tube of at least 8 French outer diameter, optionally at least 10 French, optionally at least 12 French, optionally about or at least 14 French. Such sizes of inflation can provide for rapid inflation of the inflatable region. Rapid inflation can reduce the duration needed to dilate the stent-valve, which is beneficial for the patient (e.g. it can reduced the duration of induced rapid-pacing).

The balloon-catheter characterized by any of the above optional features may be defined independently of, or in combination with, a stent-valve.

If provided, the stent-valve may comprise a stent (or stent component) and a valve (or valve component).

The stent may include an anchor region and a valve support region which are at least partly offset relative to each other in an axial direction of the stent-valve.

The valve component may include a leaflet portion. The leaflet portion may be supported by the valve support region.

With such an offset, for example: (i) at least a portion of the valve support region may not overlap axially the anchor region, and/or (ii) at least a portion of the anchor region may not overlap axially the valve support region, and/or (iii) at least a portion of the leaflet portion may not overlap axially the anchor region, and/or (iv) at least a portion of the anchor region may not overlap axially the leaflet portion.

The stent or stent-valve may optionally be of a self-expanding type.

In a further aspect, apparatus disclosed herein may comprise a balloon catheter, optionally defined according to any of the foregoing, the balloon catheter for post-implantation dilation of a stent-valve, the apparatus further comprising:
a fluid port for passage of inflation fluid into and/or out of the balloon catheter, and
a pressure relief valve for venting pressure exceeding a predetermined threshold.

Advantages of some embodiments of the invention include (i) facilitating, during post-implantation dilation of a stent-valve, application of a dilation force to an anchor region of the stent-valve (or stent) while applying less dilation to a valve (or valve support) region; and/or (ii) reducing risk of damage to a leaflet portion of a stent-valve; and/or (ii) providing a sufficiently long inflatable region to provide stability during inflation.

Non-limiting embodiments are now described by way of example only with reference to the accompanying drawing.

### Brief Description of the Drawing

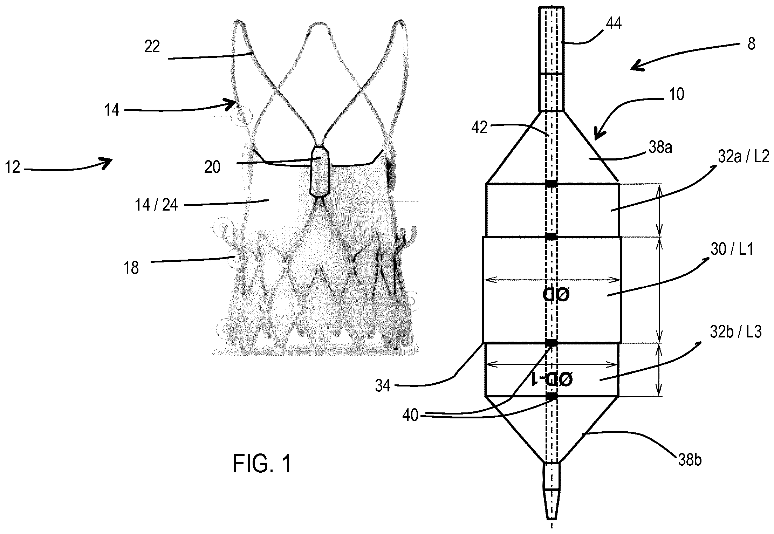

Fig. 1 is a schematic side view of a distal end of a first example of dilation balloon catheter intended for post-implantation dilation of a stent valve.

### Detailed Description of Preferred Embodiments

Referring to Fig. 1, a distal end of a balloon catheter 8 is illustrated, including an inflatable region in the form of dilation balloon 10, intended for a stent-valve 12.

In the orientation shown in Fig. 1, the balloon catheter 8 includes a distal tip (lowermost in Fig. 1), and a tube 44 extending proximally away (upwardly away in Fig. 1) from the dilation balloon 10 towards a proximal port (not shown) of the catheter 8. The catheter 8 may be illustrated when introduced in a retrograde direction with respect to an aortic stent-valve 12. However, it will be appreciated that the direction of the catheter 8 could be reversed with respect to the stent-valve 12, for example, if introduced in an antegrade direction.

In order to better appreciate certain advantages of the balloon catheter 8, some details of the stent-valve 12 will first be described. However, it is emphasized that this is merely by way of example, and that the stent-valve 12 may take a variety of other shapes and forms without detracting from the advantageous shape of the balloon catheter 8. Referring to Fig. 1, the illustrated stent valve 12 generally comprises a stent-component 14 and a valve component 16. The stent component 14 may comprise an anchor region 18 and a valve support region 20. Optionally, the stent component may further include one or more auxiliary structures 22 (e.g. stabilization arches for orientating the stent-valve during implantation). The anchor region 18 may have various shapes and configurations. In the illustrated form, the anchor region 18 may comprise opposed crowns, but a generally cylindrical or non-sculpted shape may also be used as desired. The valve component 16 may comprise at least a leaflet portion 24 supported generally by the valve support region 20 of the stent component 14.

The anchor region 18 may be configured generally to fit within or at the native annulus of a defective or stenosed heart valve. The valve support region 20 may be at least partially offset from the anchor region 18 in an axial direction, such that (i) at least a portion of the valve support region does not overlap axially the anchor region 18, and/or (ii) at least a portion of the anchor region 18 does not overlap axially the valve support region 20, and/or (iii) at least a portion of the leaflet portion 24 does not overlap axially the anchor region 18, and/or (iv) at least a portion of the anchor region 18 does not overlap axially the leaflet portion 24.

The leaflet portion 24 may be referred as being "supra-annular" and/or located above the native annulus (above being, for example, partly in the ascending aorta in the case of a stent-valve for the aortic valve position).

The stent valve 12 may be configured for delivery in a collapsed or compressed condition, for example, by a delivery catheter. The stent valve 12 may be configured to be deployed to an expanded or deployed condition for implantation. The stent-valve 12 may be configured to transition from the collapsed condition to the expanded condition by any suitable means, typically non-balloon-expansion. For example, the stent-component 14 may be a self-expanding type that expands to or at least towards the expanded state when a restraining force is removed. Additionally or alternatively, the delivery catheter may include an additional non-balloon expansion mechanism for applying forces to the stent component to cause expansion of the stent component.

The dilation catheter 8 with dilation balloon 10 may be used before implantation of the stent-valve 12 (e.g. pre-implantation valvuloplasty). Additionally or alternatively, the dilation catheter 8 with dilation balloon 10 may be used subsequent to initial deployment of the stent-valve, for example, if it is desired to proceed with post-implantation dilation of the stent-valve 12, for example, in order to improve seating of the stent-valve 12 at the native valve site.

In Fig. 1, the balloon 10 is shown side-by-side with the stent-valve 12 for ease of clarity. However, it will be appreciated that in use for post-implantation dilation, the dilation balloon would be inserted to pass at least partly within and/or extend through the stent-valve. The side-by-side view can illustrate more clearly the significance of the shape of the inflation balloon 10, and its relative positioning with respect to the stent-valve 12.

Viewed in one aspect, the dilation balloon 10 may generally comprise an inflatable region shaped for applying a dilation force to the anchor region 18 of the stent component 14, while applying less dilation force to the leaflet portion 24 of the valve component 16.

Additionally or alternatively, viewed in another aspect, the dilation balloon 10 may generally comprise a shape including a larger diameter (e.g. first) region 30 optionally configured for applying a dilation force to the anchor portion 18 of the stent-component 14, and at least one smaller diameter (e.g. second and/or third) region 32 (e.g. second region 32a and/or third region 32b) neighboring the larger diameter region. The terms "larger" and "smaller" are relative to each other. In the illustrated embodiment, two smaller diameter regions 32a and 32 may be provided neighboring the large diameter region 30 axially on both sides.

Optionally, the, or at least one, smaller diameter region (e.g. second region 32a) may, if provided, be configured for applying less dilation to the leaflet portion 24 of the valve component 14 than is applied by the larger diameter region 30 to the anchor portion 18.

Optionally, the, or at least one, smaller diameter region (e.g. third region 32b) may, if provided, be configured for applying less dilation to the left ventricle outflow tract (LVOT) than would be applied by the larger diameter region 30.

Additionally or alternatively, viewed in another aspect, the dilation balloon 10 may generally include a first region 30 of first diameter and first axial length (L1), and a second region 32a neighboring the first region, the second region of second diameter and second axial length (L2), the second diameter being smaller than the first diameter. Optionally, at least one, and optionally both, of the first and second regions 30 and 32a has or have a cylindrical shape.

The inflatable region may further include a third region 32b neighboring the first region 30 on an opposite side to the second region 32a, the third region 32b of third diameter and third axial length (L3), the third diameter smaller than the first diameter. Optionally the third region 32b has a cylindrical shape. All references below to the third region, or third axial length, or third diameter are intended to be optional, intended to be read only if the third region is provided, without implying that the third region is necessarily provided.

The difference in diameter between the larger diameter first region 30 and at least one smaller diameter second or third region 32a/b may be between about 1mm and about 5mm inclusive, optionally between about 1mm and about 3mm inclusive, optionally between about 1mm and about 2mm inclusive. Optionally, the difference in diameter with respect to the large diameter region 30 may be the same for each small diameter region 32.

Such a difference can provide the controlled dilation effects described above, yet still provide an elongate structure that is more stable and less liable to axial tilting than would be merely the larger diameter region 18 on its own.

The axial length L1 of the larger diameter first region 30 may be about or at least about 10mm, optionally about or at least about 14mm, optionally about or at least about 16mm, optionally about or at least about 18mm, optionally about or at least about 20mm, optionally about or at least about 22mm. Additionally or alternatively to any of the foregoing, the axial length L1 of the larger diameter region 30 may be about or less than about 30mm, optionally about or less than about 26mm, optionally about or less than about 24mm, optionally about or less than about 22mm, optionally about or less than about 20mm, optionally about or less than about 18mm. Additionally or alternatively to any of the foregoing, the axial length of the larger diameter region L1 may be between about 10mm and about 30mm, optionally between about 14mm and about 26mm, optionally between about 16mm and about 24mm inclusive, optionally between about 18mm and about 22mm inclusive, optionally about 20mm.

The axial length L2/L3 of at least one smaller diameter region 32a/32b may each be shorter individually than the larger diameter first region 30. The length or lengths L2/L3 may each be between about 5mm and about 20mm, optionally about 10mm. The lengths L2 and L3 may in some embodiments be about equal to each other.

The first region 30, the second region 32a and the third region 32b may define a generally continuous internal inflation space of the inflatable region. For example, the regions may inflate and expand substantially concurrently with each other.

The first region 30 may be a longitudinally central region of the inflatable balloon or inflatable region 10. Additionally or alternatively, the first region 30 may correspond to a maximum diameter of the inflatable balloon or region 10.

In some embodiments, an interface region 34 may be defined between the neighboring first and third regions 30 and 32a, and/or between the neighboring first and third regions 30 and 32b. The interface region may bridge a diameter difference between the respective regions.

If provided, an interface region 34 may have a relative short axial length (or axial extent), for example, less than about 5mm, optionally less than about 4mm, optionally less than about 3mm, optionally less than about 2mm, optionally less than about 1mm. Additionally or alternatively, the axial length of the interface region 34 may optionally be not greater than the diameter difference bridged by the interface region. Additionally or alternatively to any of the foregoing, the interface region 34 may correspond generally to a step-change in diameter.

The inflation balloon 10 may further include a fourth region 38a of decreasing diameter extending away from the second region 32a, and/or a fifth region 38b of decreasing diameter extending away from the third region 32b.

One or more radio-opaque markers 40 may be provided to enable precise positioning of the dilation balloon 10. In the illustrated example, four radio-opaque markers 40 are provided to indicate the axial extremities of the first region 30, second region 32a and the third region 32b. The radio-opaque markers may be provided on the inflatable balloon 10 and/or on a tube 42 connected to and/or passing through the balloon 10.

The tube 42 may be a single lumen tube or a multi-lumen tube. For example, the tube 42 may include a lumen space for receiving a guidewire for enabling the catheter 8 to be advanced along a guidewire. Additionally or alternatively, for example, the tube 42 may include an inflation lumen for inflation or deflation of the balloon 10 with a suitable inflation fluid. The inflation lumen may also be defined concentrically around the guide-wire lumen, at least in a portion 44 of the tube extending proximally away from the balloon 10.

The inflation lumen may be defined by a tube of at least 8 French outer diameter, optionally at least 10 French, optionally at least 12 French, optionally about or at least 14 French. Additionally or alternatively, the cross-sectional area of the inflation lumen (minus the space occupied by the guidewire lumen if arranged concentrically) may be at least about 1 mm², optionally at least about 2 mm², optionally at least about 3 mm², optionally at least about 4 mm², optionally at least about 5 mm², optionally at least about 6 mm², optionally at least about 7 mm², optionally at least about 8 mm², optionally at least about 9 mm², optionally at least about 10 mm², optionally at least about 11 mm², optionally at least about 12 mm², optionally at least about 13 mm², optionally at least about 14 mm², optionally at least about 15 mm². Such sizes of inflation lumen can provide for rapid inflation of the inflatable region 10. Rapid inflation can reduce the duration needed to dilate the stent-valve, which is beneficial for the patient (e.g. the duration of induced rapid-pacing may be reduced).

If desired, a pressure relief valve (not shown) may optionally be provided for venting inflation fluid pressure that exceeds a predetermined safety threshold. If provided, such a valve may ensure that, even when the balloon 10 is inflated rapidly with a poorly regulated inflation source, the inflation pressure does not exceed the safety threshold.

The balloon 10 may be made to precise tolerances to ensure the desired sizing.

The balloon 10 may be made of non-stretching balloon material, to ensure that the balloon does not expand elastically beyond its desired dimensions.

In some embodiments, a second inflatable region (not shown) may be provided having an envelope shape different from the balloon 10. The balloon 10 and the second inflatable region may be arranged one within another, to enable different inflation shapes to be achieved by selective inflation of the balloon 10 or the second inflatable region. At least one of the balloon 10 and the second inflatable region may be inflatable independently of the other.

Advantages of some embodiments of the invention include (i) facilitating, during post-implantation dilation of a stent-valve, application of a dilation force to an anchor region of the stent-valve (or stent) while applying less dilation to a valve (or valve support) region; and/or (ii) reducing risk of damage to a leaflet portion of a stent-valve; and/or (ii) providing a sufficiently long inflatable region to provide stability during inflation.

It is emphasized that the foregoing description is merely illustrative of non-limiting examples, and that many modifications, developments and equivalents may be used within the scope of the invention which is defined by the appended claims.

## Claims

1. A balloon catheter (8) for post-implantation dilation of a stent-valve (12), the balloon catheter (8) comprising an inflatable region (10) shaped to include:
a first region (30) having a cylindrical shape of first diameter and having a first axial length;
a second region (32a) neighboring the first region (30) on one side, the second region (32a) having a cylindrical shape of second diameter and a second axial length, the second diameter being smaller than the first diameter; and
a third region (32b) neighboring the first region (30) on an opposite side to the second region (32a), the third region(32b) having a cylindrical shape of third diameter and a third axial length, the third diameter being smaller than the first diameter,
**characterised in that**:
the balloon catheter further comprises four radio-opaque markers (40) which indicate the axial extremities of the first region (30), second region (32a) and third region (32 b).

2. A balloon catheter (8) according to claim 1, further comprising a fourth region (38a) of decreasing diameter extending away from the second region (32a), and a fifth region (38b) of decreasing diameter extending away from the third region (32b).

3. A balloon catheter (8) according to claim 1 or 2, wherein a difference between the first diameter and at least one of the second and third diameters is selected from: between 1mm and 5mm; between 1mm and 3mm; between 1mm and 2mm.

4. A balloon catheter (8) according to claim 1, 2 or 3, wherein the second and third diameters are about equal and/or the second and third axial lengths are about equal.

5. A balloon catheter (8) according to any preceding claim, wherein the first axial length is not smaller than at least one of the second and third axial lengths.

6. A balloon catheter (8) according to any preceding claim, wherein the first axial length is about equal to the sum of the second and third axial lengths.

7. A balloon catheter (8) according to any preceding claim, wherein (i) the first axial length is between 10mm and 30mm, and/or (ii) at least one of the second and third axial lengths is between 5mm and 20mm.

8. A balloon catheter (8) according to any preceding claim, wherein the first, second and third regions (30, 32a, 32b) define a generally continuous internal inflation space of the inflatable region.

9. A balloon catheter (8) according to any preceding claim, further comprising at least one interface region (34) at an interface between (i) the first region (30) and the second region (32a), and/or (ii) the first region (30) and the third region (32b);
wherein the interface region (34) bridges a diameter difference between the respective regions, and has an axial length of not more than 5mm.

10. A balloon catheter (8) according to claim 9, wherein the interface region (34) corresponds to a step-change in diameter.

11. A balloon catheter (8) according to any preceding claim, wherein the first region (30) (i) is a longitudinally central region of the inflatable region (10), and/or (ii) has a maximum diameter of the inflatable region (10).

12. A balloon catheter (8) according to any preceding claim, the balloon catheter (8) for post-implantation dilation of a stent-valve (12) that comprises a stent component and a valve component, the balloon catheter (8) comprising an inflatable region (10) shaped for applying a dilation force to an anchor portion of the stent component, while applying less dilation to a leaflet portion of the valve component.

13. A balloon catheter (8) according to any preceding claim, the balloon catheter (8) for post-implantation dilation of a stent-valve (12) that comprises a stent component and a valve component, the balloon catheter comprising an inflatable region (10) shaped to include a first region (30) configured for applying a dilation force to an anchor portion of the stent-component, and at least one second region (32a) for applying less dilation to a leaflet portion of the valve component, wherein the first region (30) has a larger diameter than the second region (32b).

14. A combination of:
a balloon catheter (8) as defined in any preceding claim; and
a stent-valve (12).

15. A combination as defined in claim 14, wherein the stent-valve (12) comprises:
a stent including an anchor region and a valve support region which are at least partly offset relative to each other; and
a valve component including a leaflet portion.

## Patentansprüche

1. Ballonkatheter (8) zur nach der Implantation erfolgenden Dilatation einer Stentklappe (12), wobei der Ballonkatheter (8) eine befüllbare Region (10) umfasst, die so geformt ist, dass sie folgendes einschließt:
eine erste Region (30) mit einer zylindrischen Form mit einem ersten Durchmesser und einer ersten axialen Länge;
eine zweite Region (32a) benachbart zu der ersten Region (30) auf einer Seite, wobei die zweite Region (32a) eine zylindrische Form mit einem zweiten Durchmesser und einer zweiten axialen Länge aufweist, wobei der zweite Durchmesser kleiner als der erste Durchmesser ist; und
eine dritte Region (32b) benachbart zu der ersten Region (30) auf einer der zweiten Region (32a) gegenüber liegenden Seite, wobei die dritte Region (32b) eine zylindrische Form mit einem dritten Durchmesser und einer dritten axialen Länge aufweist, wobei der dritte Durchmesser kleiner als der erste Durchmesser ist,
**dadurch gekennzeichnet, dass**
der Ballonkatheter des Weiteren vier strahlenundurchlässige Marker (40) umfasst, welche die axialen Extremitäten der ersten Region (30), zweiten Region (32a) und dritten Region (32b) angeben.

2. Ballonkatheter (8) nach Anspruch 1, des Weiteren umfassend eine vierte Region (38a) mit abnehmendem Durchmesser, die sich von der zweiten Region (32a) weg erstreckt, und eine fünfte Region (38b) mit abnehmendem Durchmesser, die sich von der dritten Region (32b) weg erstreckt.

3. Ballonkatheter (8) nach Anspruch 1 oder 2, wobei ein Unterschied zwischen dem ersten Durchmesser und mindestens einem von dem zweiten und dem dritten Durchmesser ausgewählt ist aus: zwischen 1 mm und 5 mm; zwischen 1 mm und 3 mm; zwischen 1 mm und 2 mm.

4. Ballonkatheter (8) nach Anspruch 1, 2 oder 3, wobei der zweite und der dritte Durchmesser etwa gleich sind und/oder die zweite und dritte axiale Länge etwa gleich sind.

5. Ballonkatheter (8) nach einem der vorhergehenden Ansprüche, wobei die erste axiale Länge nicht kleiner als mindestens eine von der zweiten und der dritten axialen Länge ist.

6. Ballonkatheter (8) nach einem der vorhergehenden Ansprüche, wobei die erste axiale Länge etwa gleich der Summe aus der zweiten und dritten axialen Länge ist.

7. Ballonkatheter (8) nach einem der vorhergehenden Ansprüche, wobei (i) die erste axiale Länge zwischen 10 mm und 30 mm liegt, und/oder (ii) mindestens eine von der zweiten und dritten axialen Länge zwischen 5 mm und 20 mm liegt.

8. Ballonkatheter (8) nach einem der vorhergehenden Ansprüche, wobei die erste, zweite und dritte Region (30, 32a, 32b) einen allgemein kontinuierlichen inneren Befüllungsraum der befüllbaren Region definieren.

9. Ballonkatheter (8) nach einem der vorhergehenden Ansprüche, des Weiteren umfassend mindestens eine Schnittstellenregion (34) an einer Schnittstelle zwischen (i) der ersten Region (30) und der zweiten Region (32a) und/oder (ii) der ersten Region (30) und der dritten Region (32b);
wobei die Schnittstellenregion (34) eine Durchmesserdifferenz zwischen den jeweiligen Regionen überbrückt und eine axiale Länge von nicht mehr als 5 mm aufweist.

10. Ballonkatheter (8) nach Anspruch 9, wobei die Schnittstellenregion (34) einer schrittweisen Änderung des Durchmessers entspricht.

11. Ballonkatheter (8) nach einem der vorhergehenden Ansprüche, wobei die erste Region (30) (i) eine längsgerichtete Zentralregion der befüllbaren Region (10) ist, und/oder (ii) einen maximalen Durchmesser der befüllbaren Region (10) aufweist.

12. Ballonkatheter (8) nach einem der vorhergehenden Ansprüche, wobei der Ballonkatheter (8) zur nach der Implantation erfolgenden Dilatation einer Stentklappe (12) dient, die eine Stentkomponente und eine Klappenkomponente umfasst, wobei der Ballonkatheter (8) eine befüllbare Region (10) umfasst, die zum Ausüben einer Dilatationskraft auf einen Ankeranteil der Stentkomponente geformt ist, während weniger Dilatation auf einen Segelanteil der Klappenkomponente ausgeübt wird.

13. Ballonkatheter (8) nach einem der vorhergehenden Ansprüche, wobei der Ballonkatheter (8) zur nach der Implantation erfolgenden Dilatation einer Stentklappe (12) dient, die eine Stentkomponente und eine Klappenkomponente umfasst, wobei der Ballonkatheter eine befüllbare Region (10) umfasst, die geformt ist, um eine erste Region (30), die ausgestaltet ist, um eine Dilatationskraft auf einen Ankeranteil der Stentkomponente auszuüben, und mindestens eine zweite Region (32a) einzuschließen, um weniger Dilatation auf einen Segelanteil der Klappenkomponente auszuüben, wobei die erste Region (30) einen größeren Durchmesser als die zweite Region (32b) aufweist.

14. Kombination, umfassend:
einen Ballonkatheter (8) wie in einem der vorhergehenden Ansprüche definiert; und eine Stentklappe (12).

15. Kombination wie in Anspruch 14 definiert, wobei die Stentklappe (12) umfasst:
einen Stent, der eine Ankerregion und eine Klappenstützregion einschließt,
die mindestens teilweise relativ zueinander versetzt sind; und
eine Klappenkomponente, die einen Segelanteil einschließt.

## Revendications

1. Cathéter à ballonnet (8) pour une dilatation post-implantation d'une endoprothèse-valvule (12), le cathéter à ballonnet (8) comprenant une région gonflable (10) formée pour comprendre :
une première région (30) ayant une forme cylindrique d'un premier diamètre et ayant une première longueur axiale ;
une deuxième région (32a) voisine de la première région (30) sur un côté, la deuxième région (32a) ayant une forme cylindrique d'un second diamètre et une seconde longueur axiale, le second diamètre étant plus petit que le premier diamètre ; et
une troisième région (32b) voisine de la première région (30) sur un côté opposé à la deuxième région (32a), la troisième région (32b) ayant une forme cylindrique d'un troisième diamètre et une troisième longueur axiale, le troisième diamètre étant plus petit que le premier diamètre,
**caractérisé en ce que** :
le cathéter à ballonnet comprend en outre quatre marqueurs radio-opaques (40) qui indiquent les extrémités axiales de la première région (30), la deuxième région (32a) et la troisième région (32b).

2. Cathéter à ballonnet (8) selon la revendication 1, comprenant en outre une quatrième région (38a) d'un diamètre décroissant s'étendant à distance de la deuxième région (32a), et une cinquième région (38b) d'un diamètre décroissant s'étendant à distance de la troisième région (32b).

3. Cathéter à ballonnet (8) selon la revendication 1 ou 2, dans lequel une différence entre le premier diamètre et au moins un des deuxième et troisième diamètres est choisie parmi : entre 1 mm et 5 mm ; entre 1 mm et 3 mm ; entre 1 mm et 2 mm.

4. Cathéter à ballonnet (8) selon la revendication 1, 2 ou 3, dans lequel les deuxième et troisième diamètres sont à peu près égaux et/ou les deuxième et troisième longueurs axiales sont à peu près égales.

5. Cathéter à ballonnet (8) selon l'une quelconque des revendications précédentes, dans lequel la première longueur axiale n'est pas plus petite qu'au moins une parmi les deuxième et troisième longueurs axiales.

6. Cathéter à ballonnet (8) selon l'une quelconque des revendications précédentes, dans lequel la première longueur axiale est à peu près égale à la somme des deuxième et troisième longueurs axiales.

7. Cathéter à ballonnet (8) selon l'une quelconque des revendications précédentes, dans lequel (i) la première longueur axiale est entre 10 mm et 30 mm, et/ou (ii) au moins une des deuxième et troisième longueurs axiales est entre 5 mm et 20 mm.

8. Cathéter à ballonnet (8) selon l'une quelconque des revendications précédentes, dans lequel les première, deuxième et troisième régions (30, 32a, 32b) délimitent un espace de gonflage interne généralement continu de la région gonflable.

9. Cathéter à ballonnet (8) selon l'une quelconque des revendications précédentes, comprenant en outre au moins une région d'interface (34) au niveau d'une interface entre (i) la première région (30) et la deuxième région (32a), et/ou (ii) la première région (30) et la troisième région (32b) ;
dans lequel la région d'interface (34) ponte une différence de diamètre entre les régions respectives, et a une longueur axiale inférieure ou égale à 5 mm.

10. Cathéter à ballonnet (8) selon la revendication 9, dans lequel la région d'interface (34) correspond à une variation brusque du diamètre.

11. Cathéter à ballonnet (8) selon l'une quelconque des revendications précédentes, dans lequel la première région (30) est une région longitudinalement centrale de la région gonflable (10), et/ou (ii) a un diamètre maximum de la région gonflable (10).

12. Cathéter à ballonnet (8) selon l'une quelconque des revendications précédentes, le cathéter à ballonnet (8) étant destiné à une dilatation post-implantation d'une endoprothèse-valvule (12) qui comprend un élément endoprothèse et un élément valvule, le cathéter à ballonnet (8) comprenant une région gonflable (10) mise en forme pour appliquer une force de dilatation à une partie ancrage de l'élément endoprothèse, tout en appliquant une dilatation moindre à une partie feuillet de l'élément valvule.

13. Cathéter à ballonnet (8) selon l'une quelconque des revendications précédentes, le cathéter à ballonnet (8) étant destiné à une dilatation post-implantation d'une endoprothèse-valvule (12) qui comprend un élément endoprothèse et un élément valvule, le cathéter à ballonnet comprenant une région gonflable (10) mise en forme pour inclure une première région (30) configurée pour appliquer une force de dilatation à une partie ancrage de l'élément endoprothèse, et au moins une seconde région (32a) pour appliquer une dilatation moindre à une partie feuillet de l'élément valvule, la première région (30) ayant un diamètre plus grand que la seconde région (32b).

14. Combinaison de :
un cathéter à ballonnet (8) selon l'une quelconque des revendications précédentes ; et
une endoprothèse-valvule (12).

15. Combinaison selon la revendication 14, dans laquelle l' endoprothèse-valvule (12) comprend :
une endoprothèse comprenant une région d'ancrage et une région de support de valvule qui sont au moins partiellement décalées l'une par rapport à l'autre ; et
un élément valvule comprenant une partie feuillet.
